# EUROPEAN PATENT APPLICATION

(11) **EP 1 020 172 A2**
(43) Date of publication of application: **19.07.2000**
(21) Application number: 99310487.6
(22) Date of filing: 23.12.1999
(51) Int. Cl.: A61G 7/057

(54) **Anti-pressure sore materials**

(30) Priority: 23.12.1998 GB 9828595; 01.06.1999 GB 9912727
(71) Applicant: Barker, Stepen George Edward, London, Greater London SE16 3SJ (GB)
(72) Inventor: Barker, Stepen George Edward, London, Greater London SE16 3SJ (GB)
(74) Representative: King, James Bertram (GB)

(57) **Abstract**

A bed sheet 30 is formed from two superposed layers of plastics material joined by welding along side margins 31 and 32 and at each end 31a and 32a. This then forms a sealed chamber. The sides 31 and 32 may include spaced pads or eyelets 33 for securing straps. The central part of the sheet 30 has a matrix of circular weld zones 34 which join together opposed portions of each side of the sheet with the spaces between the welds forming air-filled passageways. A characteristic is the wide spacing between each weld zone and the relative flatness of the air filled capsules thus formed. The capsules may be air-filled and sealed but in a preferred version each capsule is connected to an adjacent neighbour by an integral air duct thus permitting air to flow and pressure to equalise between the capsules when subject to compression. For storage the capsules may be deflated and inflated as required for use through an air inlet valve 35.

## Description

This invention relates to bed, chair or other furniture covering materials, but is directed primarily to bed covering sheets intended to act as prophylaxis against the development of pressure sores. Categories of persons who may be at risk from developing pressure sores include the aged, the chronically sick and patients who are immobile (having paralysis for example) and the obese. This invention may also be used as a stand alone device to promote comfort on sitting or lying, for example on a car seat.

One of the objects of this invention is to provide a sheeting or covering material to help reduce localised pressure on a part of the body of a person being supported for example, on a chair or on a stool or for use on a bed and which may reduce the development of pressure sores. In particular the areas include the heels and over the sacrum.

According to this invention there is provided a sheet material primarily for use on or as a bedding material and comprising a first sheet or multiple sheet layer of a plastics material incorporating a plurality of air filled capsules of a particular or variable size over the surface thereof and a second layer material comprising a fibrous and fluid absorbent layer.

In one embodiment of this invention the first layer or multiple layers comprises a sheet or sheets of a plastics material of which the surface incorporates closely spaced discrete areas occupied by air filled capsules, preferably being formed integral with the sheet

In another and preferred embodiment of this invention the air filled capsules are freely interconnected such that air may flow from one capsule to another when subjected to external pressure. In a version of this construction adjacent groups of capsules may be interconnected.

The capsules may be formed between two plastic sheets, for example made from PVC, which have been overlaid and joined together in specific areas such as along weld lines forming boundaries for the capsules and at specific circular weld spots located at intervals across the surface of the sheet. The spacings of the circular welds may vary but are distanced apart such that upon inflation the sheeting or cushion does not distort unduly. The weld areas or lines may provide or include discontinuities, forming passages for the flow and circulation of air between adjacent capsules or groups of capsules. A connection or connections is included to inflate the capsules.

In a specific preferred embodiment two sheets of plastics material are overlaid and welded together through disc shaped areas arranged in a regular matrix. This leaves spaces between the welds forming interconnecting air passages

Boundary portions of the sheet, comprising wings or flanges, may include fastening means such as eyelets for securing straps.

The material forming the detachable fibrous layer may include an absorbent fibre layer or layers covered on one or both surfaces with a paper or plastics layer. In a more preferable construction the absorbent fibrous layer is connected to a fluid pervious paper or plastics layer on one surface and a fluid impervious paper or plastics layer on the other surface. The latter layer may be bonded by any means to the sheet or sheets incorporating the air-filled capsules but may be detachable.

In use, the sheet according to this invention will be laid in a customary manner on a bed or other article of furniture or support means and would be semi-disposable. By this is meant, for example, that a single piece of sheeting or cushion might be used for a single patient stay in hospital and not used again for a second patient. The material is intended primarily for use in or on hospital beds, hospice beds, nursing home beds and similar and more particularly, for beds used for intensive care units and in high dependency hospital units or coronary care units. However, the invention, albeit in a sheet or as a cushion, could be used alone and free standing.

The sheet material according to this invention has relatively low cost, for example PVC, and is easy to use and may be applied easily to any selected bed, this being in contrast to known expensive devices or bed systems which utilise low pressure air or similar active or pumped systems.

In order to enable this invention to be better understood reference will now be made to the accompanying drawings showing embodiments by way of example.

Referring to the drawings:
**Figure 1** shows a plan view of a plastic sheet incorporating air-filled capsules,
**Figure 2** shows a section through the sheet material of this invention.
**Figure 3** shows a plan view of a further embodiment of plastic sheet, and
**Figure 4** shows an end elevation of the embodiment shown in Figure 3.

The sheet material of this invention may have an overall size comparable with that of a standard bed sheet and referring to Figure 2, there is shown a cross section through a part of the sheet, this comprising basically two layers being a plastics underlayer 1 and an absorbent top layer 2. The two layers will be bonded together by suitable means such as adhesive connections or welding which may be over the entire surface or in discrete spaced locations only.

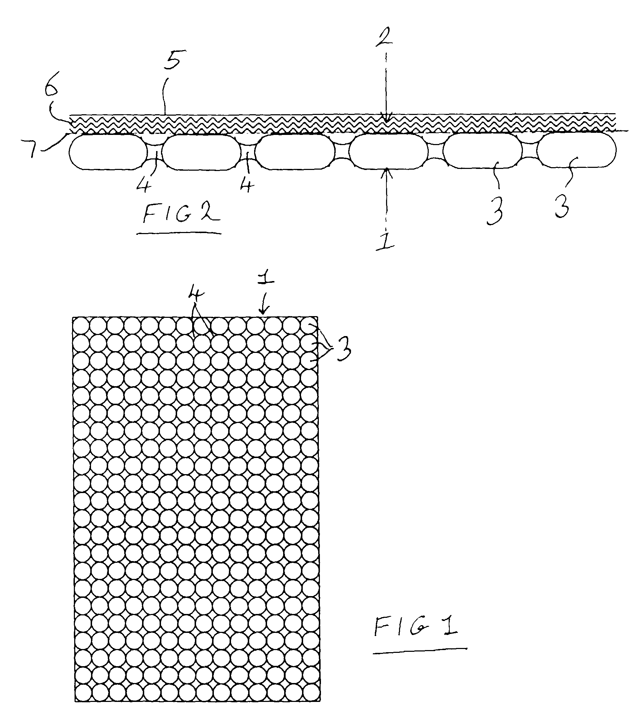

The layer 1 which in use would comprise the underside consists of a plastics material which incorporates closely spaced air filled capsules 3. Figure 1 shows in plan view the arrangement of capsules 3 on the sheet. This material may comprise a series of discrete capsules 3 as shown in Figure 2 which are interconnected by plastic webs 4. The webs 4 may provide for a air passageways to connect adjacent capsules or groups of capsules. The capsules 3 may be discrete units trapped between outer sheets which are interconnected. The sizes of these air-filled capsules may be of standard or a variable size. In an alternative construction the has a substantially flat outer surface with the air filled capsules being located on and upstanding from the other surface. In an alternative the material may comprise two juxtaposed sheets each including the air-filled capsules forming a double layer. The capsules of the one layer may be located adjacent the spaces between the capsules in the other layer. The layers may have the capsule sides facing or the non-capsule side of one layer may be adjacent the capsule side of the other layer.

The air filled capsules may each comprise a sealed unit with the volume of air therein adjusted according to requirements, that is to be soft or hard. Alternatively a plurality of capsules may be interconnected to permit air flow from one capsule to another. All adjacent capsules may be interconnected or selected capsules may be interconnected to form a group covering a selected area of the sheet. The spacing between the capsules helps determine the overall rigidity of the sheeting or cushion

In the above construction a feature is the use of a large number of capsules arranged as a regular matrix over the area of the sheet .

The absorbent material 2 may comprise an outer paper or plastics sheet material 5 which is fluid pervious and bonded to an absorbent fibrous layer 6 which is in turn connected to a further layer 7 of a fluid impervious plastics material. A suitable material for the layer 2 would be that which is sold under the trade name "Inco-pads". It will thus be seen that the essence of this invention is the provision of a fluid permeable absorbent composite layer 2 which is connected with an underlayer 1 having resiliency provided by the air filled capsules. The overall arrangement providing a sheet or cushion which is convenient to handle and which may be used on a bed or chair in order to provide comfort through absorbency of the layer 2 and cushioning of at risk parts of the body through the layer 1. The sheet may, when of a smaller size, be used on any other article of furniture of support or be used free standing.

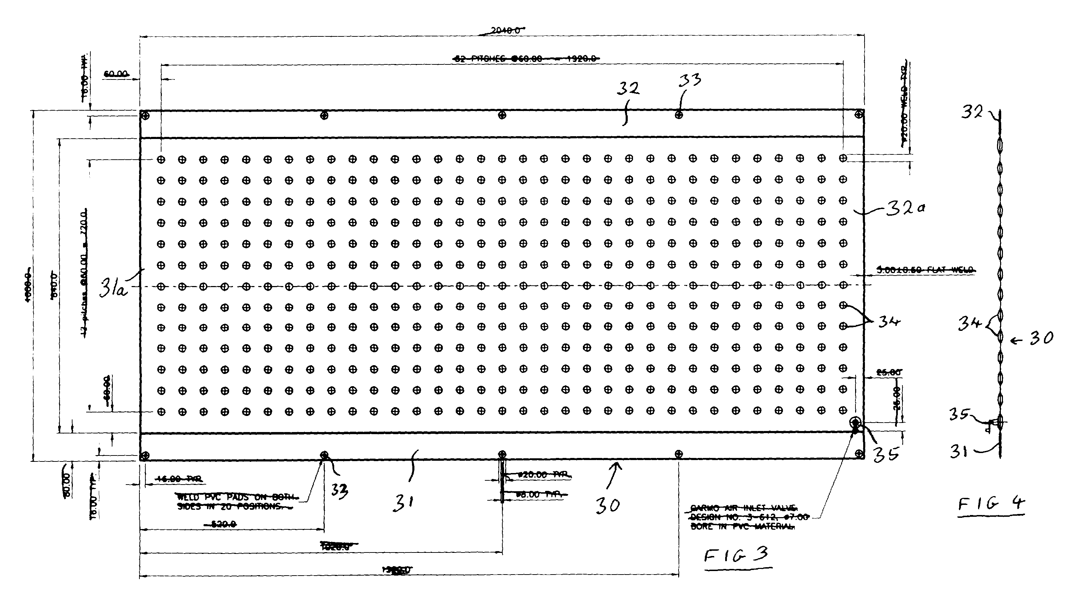

An alternative preferred construction is shown in Figures 3 and 4 comprising a bed sheet 30 of some 2 metres length and 1 metre width. The sheet is formed from two superposed layers of plastics material joined by welding along side margins 31 and 32 and at each end 31a and 32a. This then forms a sealed chamber. The sides 31 and 32 may include spaced pads or eyelets 33 for securing straps (not shown). The central part of the sheet 30 has a matrix of circular weld zones 34 which join together opposed portions of each side of the sheet with the spaces between the welds forming air-filled passageways. A characteristic of this construction is the wide spacing between each weld zone and the relative flatness of the air filled capsules thus formed. The capsules may be air-filled and sealed but in a more preferred version each capsule is connected to an adjacent neighbour by an integral air duct thus permitting air to flow and pressure to equalise between the capsules when subject to compression. For storage the capsules may be deflated and inflated as required for use through an air inlet valve 35.

Although the weld zones 34 which define the air spaces are described as circular other shapes may be used for example a cruciform configuration of weld zones may be provided which define rectangular shaped air capsules forming a checker-board configuration with discontinuities permitting air flow from one capsule to an adjacent one.

This invention also comprises, in an alternative aspect, an arrangement using the sheet material particularly in the form of Figures 3 and 4 but without the second fibrous or absorbent layer.

It will be appreciated that the overall size may be adapted to need for example to cover part only of a bed or to provide a cushion.

The sheet 30 may be formed from two overlaid sheets with the areas between discrete capsules joined by welds or adhesive with provision, where required, for air channels between adjacent capsules for example by leaving weld free tracks.

It will be appreciated that a particular feature of this invention resides in the fact that the sheet may be readily applied to an existing bed as and when required for a particular patient.

Various materials may be used for the layer 1 and in particular this may itself comprise a composite material formed from one or more layers each incorporating air filled capsules.

The absorbent material 6 of the layer 2 may comprise a bulk fibrous cotton material.

Wing portions or flanges along a side or sides of the sheet or cushion provide a means to receive tie downs to secure the device in position.

## Claims

1. A sheet material primarily for use on or as a bedding material or cushion, the sheet comprising a first sheet or multiple sheet layer of a plastics material incorporating a plurality of air filled capsules of a particular or variable size over the surface thereof and a second layer material comprising a fibrous and fluid absorbent layer.

2. A sheet in accordance with Claim 1, wherein the first layer or multiple layers comprises a sheet or sheets of a plastics material of which the surface incorporates closely spaced discrete areas occupied by air filled capsules, preferably being formed integral with the sheet.

3. A sheet according to Claim 1 or 2, wherein the air filled capsules are freely interconnected such that air may flow from one capsule to another when subjected to external pressure.

4. A sheet according to any preceding Claim, wherein adjacent groups of capsules are interconnected.

5. A sheet according to any preceding Claim, wherein the capsules are formed between two plastic sheets, for example made from PVC, which have been overlaid and joined together in specific areas such as along weld lines forming boundaries for the capsules or at specific circular weld spots located at intervals across the surface of the sheet.

6. A sheet according to any preceding Claim, wherein the spacings of the circular welds vary but are distanced apart such that upon inflation the sheeting or cushion does not distort unduly.

7. A sheet according to any preceding Claim, wherein the weld areas or lines may provide, or include discontinuities, forming passages for the flow and circulation of air between adjacent capsules or groups of capsules.

8. A sheet in accordance with any preceding Claim, wherein a connection or connections is included to inflate the capsules.

9. A sheet in accordance with any preceding Claim, wherein two sheets of plastics material are overlaid and welded together through disc shaped areas arranged in a regular matrix, the arrangement leaving spaces between the welds forming interconnecting air passages.

10. A sheet in accordance with any preceding Claim, wherein boundary portions of the sheet, comprising wings or flanges, may include fastening means such as eyelets for securing straps.

11. A sheet in accordance with any preceding Claim, wherein the material forming the detachable fibrous layer includes an absorbent fibre layer or layers covered on one or both surfaces with a paper or plastics layer.

12. A sheet in accordance with Claim 11, wherein the absorbent fibrous layer is connected to a fluid pervious paper or plastics layer on one surface and a fluid impervious paper or plastics layer on the other surface, the latter layer is preferably bonded to the sheet or sheets incorporating the air-filled capsules but may be detachable.

13. A sheet primarily for a bed the sheet being formed from two superposed layers of plastics material joined by welding along side margins and at each end to form a sealed chamber, the sides of the sheet preferably including spaced pads or eyelets for securing straps, the central part of the sheet having a matrix of circular weld zones which join together opposed portions of each side of the sheet with the spaces between the welds forming air-filled passageways.

14. A sheet in accordance with claim 13, wherein the capsules are air-filled and sealed.

15. A sheet in accordance with Claim 13, wherein each capsule is connected to an adjacent neighbour by an integral air duct thus permitting air to flow and pressure to equalise between the capsules when subject to compression.

16. A sheet in accordance with any preceding Claim 13 to 15, wherein the weld zones which define the air spaces are of a cruciform configuration which define rectangular shaped air capsules forming a checker-board configuration with discontinuities permitting air flow from one capsule to an adjacent one.

17. A sheet material or cushion substantially as described herein and illustrated with reference to Figures 1 and 2 or 3 and 4 of the drawings.
